# EUROPEAN PATENT SPECIFICATION

(11) **EP 2 694 544 B1**
(45) Date of publication and mention of the grant of the patent: **23.01.2019**
(21) Application number: 12762959.0
(22) Date of filing: 30.03.2012
(51) Int. Cl.: C07K 16/38

(54) **MONOCLONAL ANTIBODIES AGAINST TISSUE FACTOR PATHWAY INHIBITOR (TFPI)**
MONOKLONALE ANTIKÖRPER GEGEN DEN GEWEBEFAKTORINHIBITOR (TFPI)
ANTICORPS MONOCLONAUX CONTRE INHIBITEUR DE LA VOIE DU FACTEUR TISSULAIRE (TFPI)

(30) Priority: 01.04.2011 US 201161471101 P
(43) Date of publication of application: 12.02.2014
(73) Proprietor: Bayer HealthCare LLC, Whippany, NJ 07981-0915 (US)
(72) Inventor: WANG, Zhuozhi, Millbrae California 94030 (US); MURPHY, John, Berkeley California 94707 (US); MARQUARDT, Tobias, 42115 Wuppertal (DE); MOOSMAYER, Dieter, 10115 Berlin (DE)
(74) Representative: BIP Patents
(86) International application number: PCT/US2012/031538
(87) International publication number: WO 2012/135671

(56) References cited:
- WO-A1-2010/072691
- WO-A1-2011/109452
- WO-A2-2010/017196
- LWALEED BASHIR A ET AL: "Tissue factor pathway inhibitor: structure, biology and involvement in disease", JOURNAL OF PATHOLOGY, JOHN WILEY & SONS LTD, GB, vol. 208, no. 3, 1 February 2006 (2006-02-01), pages 327-339, XP002517694, ISSN: 0022-3417, DOI: 10.1002/PATH.1871 [retrieved on 2005-11-01]
- DATABASE UNIPROT [Online] 01 July 1989 XP003032876 Database accession no. P10646
- GIRARD T J ET AL: "FUNCTIONAL SIGNIFICANCE OF THE KUNITZ-TYPE INHIBITORY DOMAINS OF LIPOPROTEIN-ASSOCIATED COAGULATION INHIBITOR", NATURE, MACMILLAN JOURNALS LTD., ETC.|, vol. 338, 1 April 1989 (1989-04-01), pages 518-520, XP000857349, ISSN: 0028-0836, DOI: 10.1038/338518A0
- STEPHAN DUEBEL ED - STEFAN DÜBEL: "Handbook of Therapeutic Antibodies Chapter 6", 1 January 2007 (2007-01-01), HANDBOOK OF THERAPEUTIC ANTIBO, WILEY-VCH, WEINHEIM, PAGE(S) 119 - 144, XP007913671, ISBN: 978-3-527-31453-9

### SEQUENCE LISTING

<110> Bayer HealthCare LLC Wang, Zhuozhi Murphy, John Marquardt, Tobias Moosmayer, Dieter
<120> Monoclonal Antibodies against Tissue Factor Pathway Inhibitor (TFPI)
<130> BHC 11 5 001
<160> 9
<170> PatentIn version 3.5
<210> 1
   <211> 276
   <212> PRT
   <213> Homo Sapiens
<400> 1
   Asp Ser Glu Glu Asp Glu Glu His Thr Ile Ile Thr Asp Thr Glu Leu
   1               5                   10                  15
   Pro Pro Leu Lys Leu Met His Ser Phe Cys Ala Phe Lys Ala Asp Asp
               20                  25                  30
   Gly Pro Cys Lys Ala Ile Met Lys Arg Phe Phe Phe Asn Ile Phe Thr
           35                  40                  45
   Arg Gln Cys Glu Glu Phe Ile Tyr Gly Gly Cys Glu Gly Asn Gln Asn
       50                  55                  60
   Arg Phe Glu Ser Leu Glu Glu Cys Lys Lys Met Cys Thr Arg Asp Asn
   65                  70                  75                  80
   Ala Asn Arg Ile Ile Lys Thr Thr Leu Gln Gln Glu Lys Pro Asp Phe
                   85                  90                  95
   Cys Phe Leu Glu Glu Asp Pro Gly Ile Cys Arg Gly Tyr Ile Thr Arg
               100                 105                 110
   Tyr Phe Tyr Asn Asn Gln Thr Lys Gln Cys Glu Arg Phe Lys Tyr Gly
           115                 120                 125
   Gly Cys Leu Gly Asn Met Asn Asn Phe Glu Thr Leu Glu Glu Cys Lys
       130                 135                 140
   Asn Ile Cys Glu Asp Gly Pro Asn Gly Phe Gln Val Asp Asn Tyr Gly
   145                 150                 155                 160
   Thr Gln Leu Asn Ala Val Asn Asn Ser Leu Thr Pro Gln Ser Thr Lys
   165 170 175
   Val Pro Ser Leu Phe Glu Phe His Gly Pro Ser Trp Cys Leu Thr Pro
   180 185 190
   Ala Asp Arg Gly Leu Cys Arg Ala Asn Glu Asn Arg Phe Tyr Tyr Asn
   195 200 205
   Ser Val Ile Gly Lys Cys Arg Pro Phe Lys Tyr Ser Gly Cys Gly Gly
   210 215 220
   Asn Glu Asn Asn Phe Thr Ser Lys Gln Glu Cys Leu Arg Ala Cys Lys
   225 230 235 240
   Lys Gly Phe Ile Gln Arg Ile Ser Lys Gly Gly Leu Ile Lys Thr Lys
   245 250 255
   Arg Lys Arg Lys Lys Gln Arg Val Lys Ile Ala Tyr Glu Glu Ile Phe
   260 265 270
   Val Lys Asn Met
   275
<210> 2
   <211> 219
   <212> PRT
   <213> Homo Sapiens
<400> 2
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Val Phe Ser
   20 25 30
   Asp Gly Asn Thr Tyr Leu Asn Trp Tyr Leu Gln Lys Pro Gly Gln Ser
   35 40 45
   Pro Gln Leu Leu Ile Tyr Lys Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Gln Gln Tyr
   85 90 95
   Asp Ser Tyr Pro Leu Thr Phe Gly Gln Gly Thr Lys Val Glu Ile Lys
                   100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
                   180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Ala
       210                 215
<210> 3
   <211> 225
   <212> PRT
   <213> Homo sapiens
<400> 3
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
                   20                  25                  30
   Ser Ala Ala Trp Ser Trp Ile Arg Gln Ser Pro Gly Arg Gly Leu Glu
           35                  40                  45
   Trp Leu Gly Ile Ile Tyr Lys Arg Ser Lys Trp Tyr Asn Arg Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Trp His Ser Asp Lys His Trp Gly Phe Asp Tyr
   100 105 110
   Trp Gly Gln Gly Thr Leu Val Thr Val Ser Ser Ala Ser Thr Lys Gly
   115 120 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser
   225
<210> 4
   <211> 212
   <212> PRT
   <213> Homo sapiens
<400> 4
   Asp Ile Glu Leu Thr Gln Pro Pro Ser Val Ser Val Ala Pro Gly Gln
   1 5 10 15
   Thr Ala Arg Ile Ser Cys Ser Gly Asp Asn Leu Arg Asn Tyr Tyr Ala
   20 25 30
   His Trp Tyr Gln Gln Lys Pro Gly Gln Ala Pro Val Val Val Ile Tyr
   35 40 45
   Tyr Asp Asn Asn Arg Pro Ser Gly Ile Pro Glu Arg Phe Ser Gly Ser
   50 55 60
   Asn Ser Gly Asn Thr Ala Thr Leu Thr Ile Ser Gly Thr Gln Ala Glu
   65 70 75 80
   Asp Glu Ala Asp Tyr Tyr Cys Gln Ser Trp Asp Asp Gly Val Pro Val
                   85                  90                  95
   Phe Gly Gly Gly Thr Lys Leu Thr Val Leu Gly Gln Pro Lys Ala Ala
               100                 105                 110
   Pro Ser Val Thr Leu Phe Pro Pro Ser Ser Glu Glu Leu Gln Ala Asn
           115                 120                 125
   Lys Ala Thr Leu Val Cys Leu Ile Ser Asp Phe Tyr Pro Gly Ala Val
       130                 135                 140
   Thr Val Ala Trp Lys Gly Asp Ser Ser Pro Val Lys Ala Gly Val Glu
   145                 150                 155                 160
   Thr Thr Thr Pro Ser Lys Gln Ser Asn Asn Lys Tyr Ala Ala Ser Ser
                   165                 170                 175
   Tyr Leu Ser Leu Thr Pro Glu Gln Trp Lys Ser His Arg Ser Tyr Ser
               180                 185                 190
   Cys Gln Val Thr His Glu Gly Ser Thr Val Glu Lys Thr Val Ala Pro
           195                 200                 205
   Thr Glu Cys Ser
       210
<210> 5
   <211> 224
   <212> PRT
   <213> Homo sapiens
<400> 5
   Gln Val Glu Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser Tyr
               20                  25                  30
   Gly Met Ser Trp Val Arg Gln Ala Pro Gly Lys Gly Leu Glu Trp Val
           35                  40                  45
   Ser Ser Ile Arg Gly Ser Ser Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
   85 90 95
   Ala Arg Leu Tyr Arg Tyr Trp Phe Asp Tyr Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Lys Val Glu Pro Lys Ser Cys Asp Lys Thr His
   210 215 220
<210> 6
   <211> 219
   <212> **PRT**
   <213> Homo sapiens
<400> 6
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Val Phe Arg
   20 25 30
   Asp Gly Ile Thr Tyr Leu Asn Trp Tyr Leu Gln Lys Pro Gly Gln Ser
   35 40 45
   Pro Gln Leu Leu Ile Tyr Lys Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Gln Gln Tyr
   85 90 95
   Asp Ser Tyr Pro Leu Thr Phe Gly Gln Gly Thr Lys Val Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 7
   <211> 225
   <212> PRT
   <213> Homo sapiens
<400> 7
   Gln Val Gln Leu Gln Gln Ser Gly Pro Gly Leu Val Lys Pro Ser Gln
   1 5 10 15
   Thr Leu Ser Leu Thr Cys Ala Ile Ser Gly Asp Ser Val Ser Ser Asp
   20 25 30
   Ser Ala Ala Trp Ser Trp Ile Arg Gln Ser Pro Ser Arg Gly Leu Glu
   35 40 45
   Trp Leu Gly Ile Ile Tyr Tyr Arg Ser Lys Trp Tyr Asn Arg Tyr Ala
   50 55 60
   Val Ser Val Lys Ser Arg Ile Thr Ile Asn Pro Asp Thr Ser Lys Asn
   65 70 75 80
   Gln Phe Ser Leu Gln Leu Asn Ser Val Thr Pro Glu Asp Thr Ala Val
                   85                  90                  95
   Tyr Tyr Cys Ala Arg Trp His Ser Asp Lys His Trp Gly Phe Asp Asp
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Asn Phe Gly Thr Gln Thr Tyr Thr Cys Asn Val
           195                 200                 205
   Asp His Lys Pro Ser Asn Thr Lys Val Asp Lys Thr Val Glu Arg Lys
       210                 215                 220
   Cys
   225
<210> 8
   <211> 212
   <212> PRT
   <213> Homo sapiens
<400> 8
   Ser Tyr Glu Leu Thr Gln Pro Pro Ser Val Ser Val Ser Pro Gly Gln
   1               5                   10                  15
   Thr Ala Arg Ile Thr Cys Ser Gly Asp Asn Leu Pro Lys Tyr Tyr Ala
               20                  25                  30
   His Trp Tyr Gln Gln Lys Pro Gly Gln Ala Pro Val Val Val Ile Phe
           35                  40                  45
   Tyr Asp Val Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Met
   65                  70                  75                  80
   Asp Glu Ala Asp Tyr Tyr Cys Gln Ala Trp Trp Ser Ser Thr Pro Val
                   85                  90                  95
   Phe Gly Gly Gly Thr Lys Leu Thr Val Leu Gly Gln Pro Lys Ala Ala
               100                 105                 110
   Pro Ser Val Thr Leu Phe Pro Pro Ser Ser Glu Glu Leu Gln Ala Asn
           115                 120                 125
   Lys Ala Thr Leu Val Cys Leu Ile Ser Asp Phe Tyr Pro Gly Ala Val
       130                 135                 140
   Thr Val Ala Trp Lys Ala Asp Ser Ser Pro Val Lys Ala Gly Val Glu
   145                 150                 155                 160
   Thr Thr Thr Pro Ser Lys Gln Ser Asn Asn Lys Tyr Ala Ala Ser Ser
                   165                 170                 175
   Tyr Leu Ser Leu Thr Pro Glu Gln Trp Lys Ser His Arg Ser Tyr Ser
               180                 185                 190
   Cys Gln Val Thr His Glu Gly Ser Thr Val Glu Lys Thr Val Ala Pro
           195                 200                 205
   Thr Glu Cys Ser
       210
<210> 9
   <211> 219
   <212> PRT
   <213> Homo sapiens
<400> 9
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met Asp Trp Val Arg Gln Ala Pro Gly Lys Gly Leu Glu Trp Val
           35                  40                  45
   Ser Ser Ile Arg Gly Ser Arg Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Leu Tyr Arg Tyr Trp Phe Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Cys Ser Arg Ser Thr Ser Glu Ser Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Asn
               180                 185                 190
   Phe Gly Thr Gln Thr Tyr Thr Cys Asn Val Asp His Lys Pro Ser Asn
           195                 200                 205
   Thr Lys Val Asp Lys Thr Val Glu Arg Lys Cys
       210                 215

## Description

### Field of the embodiments

Provided are isolated monoclonal antibodies and fragments thereof that bind human tissue factor pathway inhibitor (TFPI).

### Background

Blood coagulation is a process by which blood forms stable clots to stop bleeding. The process involves a number of proenzymes and procofactors (or "coagulation factors") that are circulating in the blood. Those proenzymes and procofactors interact through several pathways through which they are converted, either sequentially or simultaneously, to the activated form. Ultimately, the process results in the activation of prothrombin to thrombin by activated Factor X (FXa) in the presence of Factor Va, ionic calcium, and platelets. The activated thrombin in turn induces platelet aggregation and converts fibrinogen into fibrin, which is then cross linked by activated Factor XIII (FXIIIa) to form a clot.

The process leading to the activation of Factor X can be carried out by two distinct pathways: the contact activation pathway (formerly known as the intrinsic pathway) and the tissue factor pathway (formerly known as the extrinsic pathway). It was previously thought that the coagulation cascade consisted of two pathways of equal importance joined to a common pathway. It is now known that the primary pathway for the initiation of blood coagulation is the tissue factor pathway.

Factor X can be activated by tissue factor (TF) in combination with activated Factor VII (FVIIa). The complex of FVIIa and its essential cofactor, TF, is a potent initiator of the clotting cascade.

The tissue factor pathway of coagulation is negatively controlled by tissue factor pathway inhibitor ("TFPI"). TFPI is a natural, FXa-dependent feedback inhibitor of the FVIIa/TF complex. It is a member of the multivalent Kunitz-type serine protease inhibitors. Physiologically, TFPI binds to activated Factor X (FXa) to form a heterodimeric complex, which subsequently interacts with the FVIIa/TF complex to inhibit its activity, thus shutting down the tissue factor pathway of coagulation. In principle, blocking TFPI activity can restore FXa and FVIIa/TF activity, thus prolonging the duration of action of the tissue factor pathway and amplifying the generation of FXa, which is the common defect in hemophilia A and B.

Indeed, some preliminary experimental evidence has indicated that blocking the TFPI activity by antibodies against TFPI normalizes the prolonged coagulation time or shortens the bleeding time. For instance, Nordfang et al. showed that the prolonged dilute prothrombin time of hemophilia plasma was normalized after treating the plasma with antibodies to TFPI (Thromb. Haemost., 1991, 66(4): 464-467). Similarly, Erhardtsen et al. showed that the bleeding time in hemophilia A rabbit model was significantly shortened by anti-TFPI antibodies (Blood Coagulation and Fibrinolysis, 1995, 6: 388-394). These studies suggest that inhibition of TFPI by anti-TFPI antibodies may be useful for the treatment of hemophilia A or B. Only polyclonal anti-TFPI antibody was used in these studies.

Using hybridoma techniques, monoclonal antibodies against recombinant human TFPI (rhTFPI) were prepared and identified (*See* Yang et al., Chin. Med. J., 1998, 111(8): 718-721). The effect of the monoclonal antibody on dilute prothrombin time (PT) and activated partial thromboplastin time (APTT) was tested. Experiments showed that anti-TFPI monoclonal antibody shortened dilute thromboplastin coagulation time of Factor IX deficient plasma. It is suggested that the tissue factor pathway plays an important role not only in physiological coagulation but also in hemorrhage of hemophilia (Yang et al., Hunan Yi Ke Da Xue Xue Bao, 1997, 22(4): 297-300).

WO 2010/017196 and WO 2010/0072691 also disclose monoclonal antibodies that bind to human tissue factor pathway inhibitor.

Accordingly, antibodies specific for TFPI are needed for treating hematological diseases and cancer.

Generally, therapeutic antibodies for human diseases have been generated using genetic engineering to create murine, chimeric, humanized or fully human antibodies. Murine monoclonal antibodies were shown to have limited use as therapeutic agents because of a short serum half-life, an inability to trigger human effector functions, and the production of human anti-mouse-antibodies (Brekke and Sandlie, "Therapeutic Antibodies for Human Diseases at the Dawn of the Twenty-first Century," Nature 2, 53, 52-62, Jan. 2003). Chimeric antibodies have been shown to give rise to human anti-chimeric antibody responses. Humanized antibodies further minimize the mouse component of antibodies. However, a fully human antibody avoids the immunogenicity associated with murine elements completely. Thus, there is a need to develop fully human antibodies to avoid the immunogenicity associated with other forms of genetically engineered monoclonal antibodies. In particular, chronic prophylactic treatment such as hemophilia treatment would be required for humanized or preferably, fully human antibodies. An anti-TFPI monoclonal antibody has a high risk of development of an immune response to the therapy if an antibody with a murine component or murine origin is used due to numerous dosing required and the long duration of therapy. For example, antibody therapy for hemophilia A may require weekly dosing for the lifetime of a patient. This would be a continual challenge to the immune system. Thus, the need exists for a fully human antibody for antibody therapy for hemophilia and related genetic and acquired deficiencies or defects in coagulation.

Therapeutic antibodies have been made through hybridoma technology described by Koehler and Milstein in "Continuous Cultures of Fused Cells Secreting Antibody of Predefined Specificity," Nature 256, 495-497 (1975). Fully human antibodies may also be made recombinantly in prokaryotes and eukaryotes. Recombinant production of an antibody in a host cell rather than hybridoma production is preferred for a therapeutic antibody. Recombinant production has the advantages of greater product consistency, likely higher production level, and a controlled manufacture that minimizes or eliminates the presence of animal-derived proteins. For these reasons, it is desirable to have a recombinantly produced monoclonal anti-TFPI antibody.

In addition, because TFPI binds to activated Factor X (FXa) with high affinity, an effective anti-TFPI antibody should have a comparable affinity. Thus, it is desirable to have an anti-TFPI antibody which has binding affinity which can compete with TFPI/FXa binding.

### Summary

Monoclonal antibodies having specific binding to a specific epitope of human tissue factor pathway inhibitor (TFPI) are provided. Also provided are polynucleotides which encode the anti-TFPI monoclonal antibodies. Pharmaceutical compositions comprising the anti-TFPI monoclonal antibodies and methods of treatment of genetic and acquired deficiencies or defects in coagulation such as hemophilia A and B are also provided.

### Brief description of the drawings

Figure 1 depicts complex formation of Fab B and TFPI Kunitz domain 1+2 by size exclusion analysis.
Figure 2 depicts two cartoon representations showing the interaction between human tissue factor pathway inhibitor and an antibody thereof (Fab B) at a first angle and at another angle rotated 90 degrees relative to the first angle. Fab B with denoted variable light (V_{L}) and heavy (V_{H}) domains is shown in the lower part of the figure (shaded in grey). TFPI Kunitz domain 1 (KD1) is shown in white and TFPI Kunitz domain 2 (KD2) is shown in black.
Figure 3 depicts key epitope residues Asp31 (D31), Asp32 (D32), Pro34 (P34), Lys36 (K36), Glu60 (E60), Cys35-Cys59 disulfide bridge, and binding of Kunitz domain 1 TFPI at the Fab B surface. Also shown, but not enumerated, is the binding of Kunitz domain 2.
Figure 4 depicts two angles of view of binding and interaction of epitope residues Glu100 (E100), Glu101 (E101), Pro103 (P103), Ile105 (1105), Arg107 (R107), Tyr109 (Y109) of Kunitz domain 2 with Fab B. Arg107 interacts with Gly33 (G33) and Cys35 (C35) of Kunitz domain 1.
Figure 5 depicts a superposition of TFPI - Fab B complex and a complex of BPTI, factor VIIa and tissue factor, and shows exclusion of simultaneous binding of TFPI to factor VIIa/tissue factor and Fab B. Steric hindrance of Fab B and factor VIIa, and Fab B and tissue factor are indicated by arrows.
Figure 6 depicts a superposition of TFPI - Fab B complex and a trypsin bound Kunitz domain 2 and shows exclusion of simultaneous binding of TFPI to factor Xa and Fab B. Steric hindrance of Fab B and trypsin, and Fab B bound Kunitz domain 1 and trypsin are indicated.
Figure 7 depicts (A) a sequence alignment of light and heavy chains of Fab B (SEQ ID NOs: 4 and 5) and Fab D (SEQ ID NOs: 8 and 9) and (B) a superposition of TFPI - Fab B X-ray structure with homology models of Fab D. (A) paratope residues are in bold text and highlighted. Paratope residues which differ in Fab B and Fab D are marked with asterisk. (B) Kunitz domain 1 (KD1) and Kunitz domain 2 (KD2) are shown as light grey and black cartoon, respectively. The Fab structures are shown as grey ribbon. Paratope residues which differ in Fab B and Fab D are shown as sticks.

### Detailed description

### Definitions

The term "tissue factor pathway inhibitor" or "TFPI" as used herein refers to any variant, isoform and species homolog of human TFPI that is naturally expressed by cells. In a preferred embodiment of the invention, the binding of an antibody of the invention to TFPI reduces the blood clotting time.

As used herein, an "antibody" refers to a whole antibody and any antigen binding fragment (i.e., "antigen-binding portion") or single chain thereof. The term includes a full-length immunoglobulin molecule (e.g., an IgG antibody) that is naturally occurring or formed by normal immunoglobulin gene fragment recombinatorial processes, or an immunologically active portion of an immunoglobulin molecule, such as an antibody fragment, that retains the specific binding activity. Regardless of structure, an antibody fragment binds with the same antigen that is recognized by the full-length antibody. For example, an anti-TFPI monoclonal antibody fragment binds to an epitope of TFPI. The antigen-binding function of an antibody can be performed by fragments of a full-length antibody. Examples of binding fragments encompassed within the term "antigen-binding portion" of an antibody include (i) a Fab fragment, a monovalent fragment consisting of the V_{L}, V_{H}, C_{L} and C_{H1} domains; (ii) a F(ab')₂ fragment, a bivalent fragment comprising two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment (Ward et al., (1989) Nature 341:544-546), which consists of a V_{H} domain; (vi) an isolated complementarity determining region (CDR); (vii) minibodies, diaboidies, triabodies, tetrabodies, and kappa bodies (see, e.g. Ill et al., Protein Eng 1997;10:949-57); (viii) camel IgG; and (ix) IgNAR . Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are analyzed for utility in the same manner as are intact antibodies.

Furthermore, it is contemplated that an antigen binding fragment may be encompassed in an antibody mimetic. The term "antibody mimetic" or "mimetic" as used herein is meant a protein that exhibits binding similar to an antibody but is a smaller alternative antibody or a non-antibody protein. Such antibody mimetic may be comprised in a scaffold. The term "scaffold" refers to a polypeptide platform for the engineering of new products with tailored functions and characteristics.

The term "epitope" refers to the area or region of an antigen to which an antibody specifically binds or interacts, which in some embodiments indicates where the antigen is in physical contact with the antibody. Conversely, the term "paratope" refers to the area or region of the antibody on which the antigen specifically binds. Epitopes characterized by competition binding are said to be overlapping if the binding of the corresponding antibodies are mutually exclusive, i.e. binding of one antibody excludes simultaneous binding of another antibody. The epitopes are said to be separate (unique) if the antigen is able to accommodate binding of both corresponding antibodies simultaneously.

The term "competing antibodies," as used herein, refers to antibodies that bind to about, substantially or essentially the same, or even the same, epitope as an antibody against TFPI as described herein. "Competing antibodies" include antibodies with overlapping epitope specificities. Competing antibodies are thus able to effectively compete with an antibody as described herein for binding to TFPI. Preferably, the competing antibody can bind to the same epitope as the antibody described herein. Alternatively viewed, the competing antibody has the same epitope specificity as the antibody described herein.

As used herein, the terms "inhibits binding" and "blocks binding" (e.g., referring to inhibition/blocking of binding of TFPI ligand to TFPI) are used interchangeably and encompass both partial and complete inhibition or blocking. Inhibition and blocking are also intended to include any measurable decrease in the binding affinity of TFPI to a physiological substrate when in contact with an anti-TFPI antibody as compared to TFPI not in contact with an anti-TFPI antibody, e.g., the blocking of the interaction of TFPI with factor Xa or blocking the interaction of a TFPI-factor Xa complex with tissue factor, factor VIIa or the complex of tissue factor/factor VIIa by at least about 10%, 20%, 30%, 40%, 50%, 60%, 70%, 80%, 90%, 95%, 96%, 97%, 98%, 99%, or 100%.

The terms "monoclonal antibody" or "monoclonal antibody composition" as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo).

An "isolated antibody," as used herein, is intended to refer to an antibody which is substantially free of other antibodies having different antigenic specificities (e.g., an isolated antibody that binds to TFPI is substantially free of antibodies that bind antigens other than TFPI). An isolated antibody that binds to an epitope, isoform or variant of human TFPI may, however, have cross-reactivity to other related antigens, e.g., from other species (e.g., TFPI species homo logs). Moreover, an isolated antibody may be substantially free of other cellular material and/or chemicals.

As used herein, "specific binding" refers to antibody binding to a predetermined antigen. Typically, the antibody binds with an affinity of at least about 10⁵ M⁻¹ and binds to the predetermined antigen with an affinity that is higher, for example at least two-fold greater, than its affinity for binding to an irrelevant antigen (e.g., BSA, casein) other than the predetermined antigen or a closely-related antigen. The phrases "an antibody recognizing an antigen" and "an antibody specific for an antigen" are used interchangeably herein with the term "an antibody which binds specifically to an antigen."

As used herein, the term "high affinity" for an IgG antibody refers to a binding affinity of at least about 10⁷M⁻¹, in some embodiments at least about 10⁸M⁻¹, in some embodiments at least about 10⁹M⁻¹, 10¹⁰M⁻¹, 10¹¹M⁻¹ or greater, e.g., up to 10¹³M⁻¹ or greater. However, "high affinity" binding can vary for other antibody isotypes. For example, "high affinity" binding for an IgM isotype refers to a binding affinity of at least about 1.0 x 10⁷M⁻¹. As used herein, "isotype" refers to the antibody class (e.g., IgM or IgG1) that is encoded by heavy chain constant region genes.

"Complementarity-determining region" or "CDR" refers to one of three hypervariable regions within the variable region of the heavy chain or the variable region of the light chain of an antibody molecule that form the N-terminal antigen-binding surface that is complementary to the three-dimensional structure of the bound antigen. Proceeding from the N-terminus of a heavy or light chain, these complementarity-determining regions are denoted as "CDR1," "CDR2," and "CDR3," respectively. CDRs are involved in antigen-antibody binding, and the CDR3 comprises a unique region specific for antigen-antibody binding. An antigen-binding site, therefore, may include six CDRs, comprising the CDR regions from each of a heavy and a light chain V region.

As used herein, "conservative substitutions" refers to modifications of a polypeptide that involve the substitution of one or more amino acids for amino acids having similar biochemical properties that do not result in loss of a biological or biochemical function of the polypeptide. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine), and aromatic side chains (e.g., tyrosine, phenylalanine, tryptophan, histidine). It is envisioned that the antibodies of the present invention may have conservative amino acid substitutions and still retain activity.

For nucleic acids and polypeptides, the term "substantial homology" indicates that two nucleic acids or two polypeptides, or designated sequences thereof, when optimally aligned and compared, are identical, with appropriate nucleotide or amino acid insertions or deletions, in at least about 80% of the nucleotides or amino acids, usually at least about 85%, preferably about 90%, 91%, 92%, 93%, 94%, or 95%, more preferably at least about 96%, 97%, 98%, 99%, 99.1%, 99.2%, 99.3%, 99.4%, or 99.5% of the nucleotides or amino acids. Alternatively, substantial homology for nucleic acids exists when the segments will hybridize under selective hybridization conditions to the complement of the strand. The invention includes nucleic acid sequences and polypeptide sequences having substantial homology to the specific nucleic acid sequences and amino acid sequences recited herein.

The percent identity between two sequences is a function of the number of identical positions shared by the sequences (i.e., % homology = # of identical positions / total # of positions x 100), taking into account the number of gaps, and the length of each gap, which need to be introduced for optimal alignment of the two sequences. The comparison of sequences and determination of percent identity between two sequences can be accomplished using a mathematical algorithm, such as without limitation the AlignX™ module of VectorNTI™ (Invitrogen Corp., Carlsbad, CA). For AlignX™, the default parameters of multiple alignment are: gap opening penalty: 10; gap extension penalty: 0.05; gap separation penalty range: 8; % identity for alignment delay: 40. (further details found at http://www.invitrogen.com/site/us/en/home/LINNEA-Online-Guides/LINNEA-Communities/Vector-NTI-Community/Sequence-analysis-and-data-management-software-for-PCs/AlignX-Module-for-Vector-NTI-Advance.reg.us.html).

Another method for determining the best overall match between a query sequence (a sequence of the present invention) and a subject sequence, also referred to as a global sequence alignment, can be determined using the CLUSTALW computer program (Thompson et al., Nucleic Acids Research, 1994, 2(22): 4673-4680), which is based on the algorithm of Higgins et al., (Computer Applications in the Biosciences (CABIOS), 1992, 8(2): 189-191). In a sequence alignment the query and subject sequences are both DNA sequences. The result of said global sequence alignment is in percent identity. Preferred parameters used in a CLUSTALW alignment of DNA sequences to calculate percent identity via pairwise alignments are: Matrix = IUB, k-tuple = 1, Number of Top Diagonals = 5, Gap Penalty = 3, Gap Open Penalty = 10, Gap Extension Penalty = 0.1. For multiple alignments, the following CLUSTALW parameters are preferred: Gap Opening Penalty = 10, Gap Extension Parameter = 0.05; Gap Separation Penalty Range = 8; % Identity for Alignment Delay = 40.

The nucleic acids may be present in whole cells, in a cell lysate, or in a partially purified or substantially pure form. A nucleic acid is "isolated" or "rendered substantially pure" when purified away from other cellular components with which it is normally associated in the natural environment. To isolate a nucleic acid, standard techniques such as the following may be used: alkaline/SDS treatment, CsCl banding, column chromatography, agarose gel electrophoresis and others well known in the art.

### Monoclonal Antibodies that bind to specific epitopes of TFPI

Provided herein are monoclonal antibodies with specific binding to human TFPI as shown in SEQ ID NO:1. In some embodiments, the anti-TFPI monoclonal antibodies inhibit binding of a TFPI ligand to TFPI. Thus, in some embodiments, the anti-TFPI monoclonal antibodies may inhibit activity of TFPI.

Provided is an isolated monoclonal antibody that binds to an epitope of human tissue factor pathway inhibitor (SEQ ID NO:1), wherein said epitope comprises one or more residues of Kunitz domain 2. The isolated monoclonal according to the invention comprises the light chain as shown in SEQ ID NO:4 and the heavy chain as shown in SEQ ID NO:5. In some aspects of the disclosure, it is also contemplated that the isolated monoclonal antibody may comprise a light chain or heavy chain with substantial homology to those provided. For example, the isolated monoclonal antibody comprising substantial homology may comprise one or more conservative substitutions.

In some aspects of the disclosure, provided is an isolated monoclonal antibody that binds to an epitope of human tissue factor pathway inhibitor (SEQ ID NO:1), wherein said epitope comprises one or more residues selected from Glu100, Glu101, Gly104, Ile105, Cys106, Arg107, Gly108, Tyr109, Lys126, Gly128, of SEQ ID NO:1 and combinations thereof.

In some embodiments, the epitope comprises residue Glu100 of SEQ ID NO:1. In some embodiments, the epitope comprises residue Glu101 of SEQ ID NO:1. In some embodiments, the epitope comprises residue Gly104 of SEQ ID NO:1. In some embodiments, the epitope comprises residue Ile105 of SEQ ID NO:1. In some embodiments, the epitope comprises residue Cys106 of SEQ ID NO:1. In some embodiments, the epitope comprises residue Arg107 of SEQ ID NO:1. In some embodiments, the epitope comprises residue Gly108 of SEQ ID NO:1. In some embodiments, the epitope comprises residue Tyr109 of SEQ ID NO:1. In some embodiments, the epitope comprises residue Lys126 of SEQ ID NO:1. In some embodiments, the epitope comprises residue Gly128 of SEQ ID NO:1

In some embodiments, the epitope further comprises one or more residues selected from Glu100, Glu101, Asp102, Pro103, Gly104, Ile105, Cys106, Arg107, Gly108, Tyr109, Lys126, Gly128, Gly129, Cys130, Leu131, Gly132, and Asn133 of SEQ ID NO:1. In some embodiments, the epitope comprises residue Ile105 of SEQ ID NO:1. And in some embodiments, the epitope further comprises one or more residues selected from Glu100, Glu101, Asp102, Pro103, Gly104, Ile105, Cys106, Arg107, Gly108, Tyr109, Lys126, Gly128, Gly129, Cys130, Leu131, Gly132, and Asn133 of SEQ ID NO:1.

Also provided is an isolated monoclonal antibody that binds to an epitope of human tissue factor pathway inhibitor (SEQ ID NO:1), wherein said epitope comprises one or more residues of Kunitz domain 1 and one or more residues of Kunitz domain 2.

In some embodiments, the residues of Kunitz domain 1 comprise one or more residues selected from Asp31, Asp32, Gly33, Pro34, Cys35, Lys36, Cys59, Glu60 and Asn62 of SEQ ID NO:1 and combinations thereof. In some embodiments, the residue of Kunitz domain 1 comprises residue Asp31 of SEQ ID NO:1. In some embodiments, the residue of Kunitz domain 1 comprises residue Asp32 of SEQ ID NO:1. In some embodiments, the residue of Kunitz domain 1 comprises residue Gly33 of SEQ ID NO:1. In some embodiments, the residue of Kunitz domain 1 comprises residue Pro34 of SEQ ID NO:1. In some embodiments, the residue of Kunitz domain 1 comprises residue Cys35 of SEQ ID NO:1. In some embodiments, the residue of Kunitz domain 1 comprises residue Lys36 of SEQ ID NO:1. In some embodiments, the residue of Kunitz domain 1 comprises residue Cys59 of SEQ ID NO:1. In some embodiments, the residue of Kunitz domain 1 comprises residue Glu60 of SEQ ID NO:1. In some embodiments, the residue of Kunitz domain 1 comprises residue Asn62 of SEQ ID NO:1.

In some embodiments, the residues of Kunitz domain 1 comprise residues Pro34 and Glu60 of SEQ ID NO:1. In some embodiments, the residues of Kunitz domain 1 comprise residues Pro34 and Lys36 of SEQ ID NO:1. In some embodiments, the residues of Kunitz domain 1 comprise residues Pro34, Lys36 and Glu60 of SEQ ID NO:1.

In some embodiments, the residues of Kunitz domain 2 comprise one or more residues selected from Glu100, Glu101, Pro103, Gly104, Ile105, Cys106, Arg107, Gly108, Tyr109, Phe114, Asn116, Glu123, Arg124, Lys126, Tyr127 and Gly128 of SEQ ID NO:1 and combinations thereof. In some embodiments, the residue of Kunitz domain 2 comprises residue Glu100 of SEQ ID NO:1. In some embodiments, the residue of Kunitz domain 2 comprises residue Glu101 of SEQ ID NO:1. In some embodiments, the residue of Kunitz domain 2 comprises residue Pro103 of SEQ ID NO:1. In some embodiments, the residue of Kunitz domain 2 comprises residue Gly104 of SEQ ID NO:1. In some embodiments, the residue of Kunitz domain 2 comprises residue Ile105 of SEQ ID NO:1. In some embodiments, the residue of Kunitz domain 2 comprises residue Cys106 of SEQ ID NO:1. In some embodiments, the residue of Kunitz domain 2 comprises residue Arg107 of SEQ ID NO:1. In some embodiments, the residue of Kunitz domain 2 comprises residue Gly108 of SEQ ID NO:1. In some embodiments, the residue of Kunitz domain 2 comprises residue Tyr109 of SEQ ID NO:1. In some embodiments, the residue of Kunitz domain 2 comprises residue Phe114 of SEQ ID NO:1. In some embodiments, the residue of Kunitz domain 2 comprises residue Asn116 of SEQ ID NO:1. In some embodiments, the residue of Kunitz domain 2 comprises residue Glu123 of SEQ ID NO:1. In some embodiments, the residue of Kunitz domain 2 comprises residue Arg124 of SEQ ID NO:1. In some embodiments, the residue of Kunitz domain 2 comprises residue Lys126 of SEQ ID NO:1. In some embodiments, the residue of Kunitz domain 2 comprises residue Tyr127 of SEQ ID NO:1.

In some embodiments, the residue of Kunitz domain 2 comprises residues Arg107 and Glu101 of SEQ ID NO:1. In some embodiments, the residue of Kunitz domain 2 comprises residues Arg107 and Tyr109 of SEQ ID NO:1. In some embodiments, the residue of Kunitz domain 2 comprises residues Arg107, Glu101 and Tyr109 of SEQ ID NO:1. In some embodiments, the residue of Kunitz domain 2 comprises residue Gly128 of SEQ ID NO:1.

In some embodiments, the residue of Kunitz domain 2 may additionally comprise one or more residues selected from Asp102, Gly129, Cys130, Leu131, Gly132, and Asn133 of SEQ ID NO:1 and combinations thereof.

In some aspects of the disclosure, the isolated monoclonal antibody comprises a residue of Kunitz domain 1 which comprises one or more residues selected from Asp31, Asp32, Gly33, Pro34, Cys35, Lys36, Cys59, Glu60 and Asn62; and a residue of Kunitz domain 2 which comprises one or more residues selected from Glu100, Glu101, Pro103, Gly104, Ile105, Cys106, Arg107, Gly108, Tyr109, Phe114, Asn116, Glu123, Arg124, Lys126, Tyr127 and Gly128.

Also provided is an isolated monoclonal antibody that binds to an epitope of human tissue factor pathway inhibitor (SEQ ID NO:1), wherein said epitope comprises two amino acid loops linked by a disulfide bridge between residues Cys35 and Cys59 of SEQ ID NO:1. In some aspects, the epitope further comprises one or more residues of Kunitz domain 1 and one or more residues of Kunitz domain 2. In some embodiments, the residue of Kunitz domain 1 comprises one or more residues selected from Asp31, Asp32, Gly33, Pro34, Cys35, Lys36, Cys59, Glu60, and Asn62 of SEQ ID NO:1. In some aspects, the residue of Kunitz domain 2 comprises one or more residues selected from Glu100, Glu101, Pro103, Gly104, Ile105, Cys106, Arg107, Gly108, Tyr109, Phe114, Asn116, Glu123, Arg124, Lys126, Tyr127 and Gly128 of SEQ ID NO:1.

Also provided are antibodies which can compete with any of the antibodies described herein for binding to TFPI. For example, an antibody that binds to the same epitope as the antibodies described herein will be able to effectively compete for binding of TFPI. In some aspects of the disclosure, provided is an isolated monoclonal antibody that binds to TFPI, wherein the isolated monoclonal antibody is competitive with any of the isolated monoclonal antibodies described herein. In some aspects of the disclosure, the antibody is competitive with an antibody having a light chain as shown in SEQ ID NO:4. In some aspects of the disclosure, the antibody is competitive with an antibody having a heavy chain as shown in SEQ ID NO:5. In some aspects of the disclosure, the antibody is competitive with an antibody having a light chain as shown in SEQ ID NO:4 and a heavy chain as shown in SEQ ID NO:5.

Also provided are bispecific antibodies which can compete with any of the antibodies described herein for binding to TFPI. For example, such bispecific antibody may bind to one or more epitopes described above.

The antibody may be species specific or may cross react with multiple species. In some embodiments, the antibody may specifically react or cross react with TFPI of human, mouse, rat, guinea pig, rabbit, monkey, pig, dog, cat or other mammalian species.

The antibody may be of any of the various classes of antibodies, such as without limitation an IgG1, an IgG2, an IgG3, an IgG4, an IgM, an IgA1, an IgA2, a secretory IgA, an IgD, and an IgE antibody.

### Nucleic Acids, Vectors and Host Cells

Although provided are amino acid sequences of the monoclonal antibodies, it is contemplated that nucleic acid sequences can be designed to encode any of these monoclonal antibodies. Such polynucleotides may encode encode a light chain or a heavy chain of the anti-TFPI antibody. In some embodiments, such polynucleotides may encode both the light chain and heavy chain of the anti-TFPI antibody separated by a degradeable linkage. Further, above mentioned antibodies can be produced using expression vectors comprising the isolated nucleic acid molecules encoding any of the monoclonal antibodies and host cells comprising such vectors.

### Methods of Preparing Antibodies to TFPI

The monoclonal antibody may be produced recombinantly by expressing a nucleotide sequence encoding the variable regions of the monoclonal antibody according to the embodiments of the invention in a host cell. With the aid of an expression vector, a nucleic acid containing the nucleotide sequence may be transfected and expressed in a host cell suitable for the production. Accordingly, also provided is a method for producing a monoclonal antibody that binds with human TFPI comprising:
(a) transfecting a nucleic acid molecule encoding a monoclonal antibody of the invention into a host cell,
(b) culturing the host cell so to express the monoclonal antibody in the host cell, and optionally
(c) isolating and purifying the produced monoclonal antibody, wherein the nucleic acid molecule comprises a nucleotide sequence encoding a monoclonal antibody of the present invention.

In one example, to express the antibodies, or antibody fragments thereof, DNAs encoding partial or full-length light and heavy chains obtained by standard molecular biology techniques are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vectors or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present). The light and heavy chain variable regions of the antibodies described herein can be used to create full-length antibody genes of any antibody isotype by inserting them into expression vectors already encoding heavy chain constant and light chain constant regions of the desired isotype such that the V_{H} segment is operatively linked to the C_{H} segment(s) within the vector and the V_{L} segment is operatively linked to the C_{L} segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody chain encoding genes, the recombinant expression vectors of the invention carry regulatory sequences that control the expression of the antibody chain genes in a host cell. The term "regulatory sequence" is intended to include promoters, enhancers and other expression control elements (e.g., polyadenylation signals) that control the transcription or translation of the antibody chain genes. Such regulatory sequences are described, for example, in Goeddel; Gene Expression Technology. Methods in Enzymology 185, Academic Press, San Diego, Calif. (1990). It will be appreciated by those skilled in the art that the design of the expression vector, including the selection of regulatory sequences may depend on such factors as the choice of the host cell to be transformed, the level of expression of protein desired, etc. Examples of regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (e.g., the adenovirus major late promoter (AdMLP)) and polyoma. Alternatively, nonviral regulatory sequences may be used, such as the ubiquitin promoter or β-globin promoter.

In addition to the antibody chain genes and regulatory sequences, the recombinant expression vectors may carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216, 4,634,665 and 5,179,017, all by Axel et al.). For example, typically the selectable marker gene confers resistance to drugs, such as G418, hygromycin or methotrexate, on a host cell into which the vector has been introduced. Examples of selectable marker genes include the dihydrofolate reductase (DHFR) gene (for use in dhfr- host cells with methotrexate selection/amplification) and the neo gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody.

Examples of mammalian host cells for expressing the recombinant antibodies include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, e.g., as described in R. J. Kaufman and P. A. Sharp (1982) Mol. Biol. 159:601-621), NSO myeloma cells, COS cells, HKB11 cells and SP2 cells. When recombinant expression vectors encoding antibody genes are introduced into mammalian host cells, the antibodies are produced by culturing the host cells for a period of time sufficient to allow for expression of the antibody in the host cells or secretion of the antibody into the culture medium in which the host cells are grown. Antibodies can be recovered from the culture medium using standard protein purification methods, such as ultrafiltration, size exclusion chromatography, ion exchange chromatography and centrifugation.

### Use of Partial Antibody Sequences to Express Intact Antibodies

Antibodies interact with target antigens predominantly through amino acid residues that are located in the six heavy and light chain CDRs. For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al., 1998, Nature 332:323-327; Jones, P. et al., 1986, Nature 321:522-525; and Queen, C. et al., 1989, Proc. Natl. Acad. Sci. U.S.A. 86:10029-10033). Such framework sequences can be obtained from public DNA databases that include germline antibody gene sequences. These germline sequences will differ from mature antibody gene sequences because they will not include completely assembled variable genes, which are formed by V(D)J joining during B cell maturation. It is not necessary to obtain the entire DNA sequence of a particular antibody in order to recreate an intact recombinant antibody having binding properties similar to those of the original antibody (see WO 99/45962). Partial heavy and light chain sequence spanning the CDR regions is typically sufficient for this purpose. The partial sequence is used to determine which germline variable and joining gene segments contributed to the recombined antibody variable genes. The germline sequence is then used to fill in missing portions of the variable regions. Heavy and light chain leader sequences are cleaved during protein maturation and do not contribute to the properties of the final antibody. For this reason, it is necessary to use the corresponding germline leader sequence for expression constructs. To add missing sequences, cloned cDNA sequences can be combined with synthetic oligonucleotides by ligation or PCR amplification. Alternatively, the entire variable region can be synthesized as a set of short, overlapping, oligonucleotides and combined by PCR amplification to create an entirely synthetic variable region clone. This process has certain advantages such as elimination or inclusion or particular restriction sites, or optimization of particular codons.

The nucleotide sequences of heavy and light chain transcripts are used to design an overlapping set of synthetic oligonucleotides to create synthetic V sequences with identical amino acid coding capacities as the natural sequences. The synthetic heavy and light chain sequences can differ from the natural sequences in three ways: strings of repeated nucleotide bases are interrupted to facilitate oligonucleotide synthesis and PCR amplification; optimal translation initiation sites are incorporated according to Kozak's rules (Kozak, 1991, J. Biol. Chem. 266:19867-19870); and restricted endonuclease sites are engineered upstream of the translation initiation sites.

For both the heavy and light chain variable regions, the optimized coding, and corresponding non-coding, strand sequences are broken down into 30-50 nucleotide sections at approximately the midpoint of the corresponding non-coding oligonucleotide. Thus, for each chain, the oligonucleotides can be assembled into overlapping double stranded sets that span segments of 150-400 nucleotides. The pools are then used as templates to produce PCR amplification products of 150-400 nucleotides. Typically, a single variable region oligonucleotide set will be broken down into two pools which are separately amplified to generate two overlapping PCR products. These overlapping products are then combined by PCR amplification to form the complete variable region. It may also be desirable to include an overlapping fragment of the heavy or light chain constant region in the PCR amplification to generate fragments that can easily be cloned into the expression vector constructs.

The reconstructed heavy and light chain variable regions are then combined with cloned promoter, translation initiation, constant region, 3' untranslated, polyadenylation, and transcription termination sequences to form expression vector constructs. The heavy and light chain expression constructs can be combined into a single vector, co-transfected, serially transfected, or separately transfected into host cells which are then fused to form a host cell expressing both chains.

Thus, in another aspect, the structural features of a human anti-TFPI antibody are used to create structurally related human anti-TFPI antibodies that retain the function of binding to TFPI. More specifically, one or more CDRs of the specifically identified heavy and light chain regions of the monoclonal antibodies of the invention can be combined recombinantly with known human framework regions and CDRs to create additional, recombinantly-engineered, human anti-TFPI antibodies of the invention.

### Pharmaceutical Compositions

Also provided are pharmaceutical compositions comprising therapeutically effective amounts of anti-TFPI monoclonal antibody and a pharmaceutically acceptable carrier. "Pharmaceutically acceptable carrier" is a substance that may be added to the active ingredient to help formulate or stabilize the preparation and causes no significant adverse toxicological effects to the patient. Examples of such carriers are well known to those skilled in the art and include water, sugars such as maltose or sucrose, albumin, salts such as sodium chloride, etc. Other carriers are described for example in Remington's Pharmaceutical Sciences by E. W. Martin. Such compositions will contain a therapeutically effective amount of at least one anti-TFPI monoclonal antibody. In some embodiments, such compositions may comprise a therapeutically effective amount of one or more anti-TFPI monoclonal antibodies. In some embodiments, the pharmaceutical compositions may comprise an antibody that specifically binds to Kunitz domain 1 as described above and an antibody that specifically binds to Kunitz domain 1 and 2 as describe above.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. The composition is preferably formulated for parenteral injection. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. In some cases, it will include isotonic agents, for example, sugars, polyalcohols such as mannitol, sorbitol, or sodium chloride in the composition.

Sterile injectable solutions can be prepared by incorporating the active compound in the required amount in an appropriate solvent with one or a combination of ingredients enumerated above, as required, followed by sterilization micro filtration. Generally, dispersions are prepared by incorporating the active compound into a sterile vehicle that contains a basic dispersion medium and the required other ingredients from those enumerated above. In the case of sterile powders for the preparation of sterile injectable solutions, some methods of preparation are vacuum drying and freeze-drying (lyophilization) that yield a powder of the active ingredient plus any additional desired ingredient from a previously sterile-filtered solution thereof.

### Pharmaceutical Uses

The monoclonal antibody can be used for therapeutic purposes for treating genetic and acquired deficiencies or defects in coagulation. For example, the monoclonal antibodies in the embodiments described above may be used to block the interaction of TFPI with FXa, or to prevent TFPI-dependent inhibition of the TF/FVIIa activity. Additionally, the monoclonal antibody may also be used to restore the TF/FVIIa-driven generation of FXa to bypass the insufficiency of FVIII- or FIX-dependent amplification of FXa.

The monoclonal antibodies have therapeutic use in the treatment of disorders of hemostasis such as thrombocytopenia, platelet disorders and bleeding disorders (e.g., hemophilia A, hemophilia B and hemophilia C). Such disorders may be treated by administering a therapeutically effective amount of the anti-TFPI monoclonal antibody to a patient in need thereof. The monoclonal antibodies also have therapeutic use in the treatment of uncontrolled bleeds in indications such as trauma and hemorrhagic stroke. Thus, also provided is a method for shortening the bleeding time comprising administering a therapeutically effective amount of an anti-TFPI monoclonal antibody of the invention to a patient in need thereof.

The antibodies can be used as monotherapy or in combination with other therapies to address a hemostatic disorder. For example, co-administration of one or more antibodies of the invention with a clotting factor such as factor VIIa, factor VIII or factor IX is believed useful for treating hemophilia. In one embodiment, provided is a method for treating genetic and acquired deficiencies or defects in coagulation comprising administering (a) a first amount of a monoclonal antibody that binds to human tissue factor pathway inhibitor and (b) a second amount of factor VIII or factor IX, wherein said first and second amounts together are effective for treating said deficiencies or defects. In another embodiment, provided is a method for treating genetic and acquired deficiencies or defects in coagulation comprising administering (a) a first amount of a monoclonal antibody that binds to human tissue factor pathway inhibitor and (b) a second amount of factor VIII or factor IX, wherein said first and second amounts together are effective for treating said deficiencies or defects, and further wherein factor VII is not coadministered. The invention also includes a pharmaceutical composition comprising a therapeutically effective amount of the combination of a monoclonal antibody of the invention and factor VIII or factor IX, wherein the composition does not contain factor VII. "Factor VII" includes factor VII and factor VIIa. These combination therapies are likely to reduce the necessary infusion frequency of the clotting factor. By co-administration or combination therapy is meant administration of the two therapeutic drugs each formulated separately or formulated together in one composition, and, when formulated separately, administered either at approximately the same time or at different times, but over the same therapeutic period.

The pharmaceutical compositions may be parenterally administered to subjects suffering from hemophilia A or B at a dosage and frequency that may vary with the severity of the bleeding episode or, in the case of prophylactic therapy, may vary with the severity of the patient's clotting deficiency.

The compositions may be administered to patients in need as a bolus or by continuous infusion. For example, a bolus administration of an inventive antibody present as a Fab fragment may be in an amount of from 0.0025 to 100 mg/kg body weight, 0.025 to 0.25 mg/kg, 0.010 to 0.10 mg/kg or 0.10-0.50 mg/kg. For continuous infusion, an inventive antibody present as an Fab fragment may be administered at 0.001 to 100 mg/kg body weight/minute, 0.0125 to 1.25 mg/kg/min., 0.010 to 0.75 mg/kg/min., 0.010 to 1.0 mg/kg/min. or 0.10-0.50 mg/kg/min. for a period of 1-24 hours, 1-12 hours, 2-12 hours, 6-12 hours, 2-8 hours, or 1-2 hours. For administration of an inventive antibody present as a full-length antibody (with full constant regions), dosage amounts may be about 1-10 mg/kg body weight, 2-8 mg/kg, or 5-6 mg/kg. Such full-length antibodies would typically be administered by infusion extending for a period of thirty minutes to three hours. The frequency of the administration would depend upon the severity of the condition. Frequency could range from three times per week to once every two or three weeks.

Additionally, the compositions may be administered to patients via subcutaneous injection. For example, a dose of 10 to 100 mg anti-TFPI antibody can be administered to patients via subcutaneous injection weekly, biweekly or monthly.

As used herein, "therapeutically effective amount" means an amount of an anti-TFPI monoclonal antibody or of a combination of such antibody and factor VIII or factor IX that is needed to effectively increase the clotting time in vivo or otherwise cause a measurable benefit in vivo to a patient in need. The precise amount will depend upon numerous factors, including, but not limited to the components and physical characteristics of the therapeutic composition, intended patient population, individual patient considerations, and the like, and can readily be determined by one skilled in the art.

### Examples

### Example 1. Expression and purification of recombinant TFPI (Kunitz domain 2) from E. coli.

### Expression system

The destination vector (according to Gateway nomenclature), designated pD Eco5 N, was utilized. pD Eco5 N is based on the pET-16 b (Novagen), and additionally encodes a His₁₀ and NusA tag as well as a Gateway cloning cassette for expression of the fusion protein consisting of His₁₀/NusA and the protein of interest.

A TFPI construct encoding a thrombin cleavage site fused to the N-terminus of Kunitz domain 2 (Lys93 to Phe154, reference Uniprot 10646) and the Gateway attachment sites (attB1-5#, attB2-3#, Invitrogen) was cloned into the pD Eco5 N vector resulting in the expression vector designated as pD Eco5 N TFPI KD2. The BL21 DE3 (Novagen) expression strain was utilized.

### Amino acid sequence of expressed fusion protein using pD Eco5 N TFPI KD2, 600 AA

### Sequence components

| | |
|---|---|
| His 10 tag: | MGHHHHHHHH HH |
| NusA tag: | |
| Linker/translated endonulease restriction sites: | |
| | TSGS GLE |
| Translated att-site: | |
| | TSLYKKA GS |
| Thrombin site: | LVPRGS |
| TFPI Kunitz 2 | |

### Expression

A BL21 DE3 strain transformed with pD Eco5 N #209 was grown as a pre-culture in 2x 50 mL LB medium with 200 µg/mL ampicillin for 14 h at 37 °C with an agitatation rate of 180 rpm. Next, eight shaker flasks with 400 mL Circlegrow medium (Q-Biogene), were each inoculated with 8 mL pre-culture and incubated at 37 °C, with an agitation rate of 180 rpm. At a culture density of OD600, IPTG (100 mM final concentration) was added for gene induction and further cultivated at 17 °C for 24 h with 180 rpm. The E. coli was pelleted by centrifugation (3000 g, 10 min) and stored at -80 °C.

### Purification

The pelleted E. coli mass from 3.2 L of culture was resuspended in 200 mL of lysis buffer (50 mM Tris-HCl pH 8.0, 300 mM NaCl, 10% (w/w) glycerol, 40 mM imidazol, protease inhibitor cocktail Complete EDTA-free (Roche)), homogenized in a high pressure device (Microfluidics) and afterwards the lysate was centrifuged (100.000 g, 60 min, 4 °C). Several purification steps were performed using an Äkta Explorer system. The concentrated sample was applied in an initial IMAC chromatography step to two linked 5 mL units of Hi-Trap-Sepharose HP matrix (GE). Equilibration, fusion protein binding and wash of the Hi-Trap-Sepharose HP matrix was done using Buffer A (50 mM Tris HCl pH 8.0, 300 mM NaCl, 40 mM imidazol). For elution of the NusA-TFPI fusion protein, a linear gradient of imidazol from 40 to 500 mM in Buffer B (50 mM Tris HCl pH 8.0, 150 mM NaCl) was used. The elution fractions were pooled and concentrated (by a factor of 6-7 using a Amicon ultrafiltration device) and the buffer exchanged to Tris HCl pH 8.0. The concentrated sample (6-7 mL) was further applied to size exclusion chromatoraphy using Sephacryl-100 (XK26/74) in Tris HCl pH 8.0. The fractions of the main peak containing fusion protein were pooled, concentrated by ultrafiltration (Amicon) to 5 mL volume. Thrombin (HTI) was added to the sample (ratio enzyme : fusion protein, 1:50 w/w), incubated for 5 h at 21 °C and the reaction finally stopped by PMSF (1 mM final concentration). Subsequently, a second size exclusion chromatography step (Sephacryl-100 (XK26/74) in Tris HCl pH 8.0) was performed and the peak fractions monitored by PAGE. The fractions containing the free monomeric TFPI Kunitz domain 2 were collected and concentrated (Amicon), yielding about 4 mg of product from 3.2 L E. coli culture.

### Example 2. Production of recombinant TFPI (Kunitz domain 1 + 2), expression in E. coli and its purification

### Expression system

The destination vector (according to Gateway nomenclature), designated pD Eco5 N is based on ET-16 b (Novagen). The vector also encodes a His₁₀ and NusA tag, as well as the Gateway cloning cassette for expression of the fusion protein consisting of His₁₀/NusA and the protein of interest.

A DNA construct encoding a TEV protease cleavage site fused to the N-terminus of the Kunitz domains 1 + 2 (Asp1 to Phe154, reference Uniprot 10646, mature TFPI alpha) and the Gateway attachment sites (attB1-5#, attB2-3#, Invitrogen) was cloned into the pD Eco5 N vector resulting in the expression vector designated as pD Eco5 N TFPI KD1+2. The expression strain used was BL21 DE3 (Novagen).

### Amino acid sequence of expressed fusion protein using pD Eco5 N TFPI KD1+2, 600 AA

### Sequence components

| | |
|---|---|
| **His 10 tag:** | MGHHHHHHHH HH |
| **NusA tag:** | |
| **Linker/translated endonulease restriction sites:** | TSGS GLE |
| **Translated att-site:** | TSLYKKA GS |
| **TEV site:** | DYDIPTTENLYFQ |
| **TFPI Kunitz 1+2** | |

### Expression

A BL21 DE3 strain transformed with pD Eco5 N TFPI KD1+2 was grown as a pre-culture in 2x 100 mL of LB medium with 200 µg/mL ampicillin for 14 h at 37 °C with agitation of 180 rpm. A Bioreactor (Sartorius Stedim Biotech) with 10 L culture volume (LB medium, 200 µg/mL ampicillin) was inoculated with 200 mL pre-culture and incubated at 37 °C, with agitation of 150 rpm. At a culture density of OD600, IPTG (isopropyl *β*-D-thiogalactoside) was added to a final concentration of 100 mM for gene induction and further cultivated at 17 °C for 24 h with a pO2 minimum level of 50% and an agitation rate of 180 - 800 rpm. The E. coli was pelleted by centrifugation (3000 g, 10 min) and stored at -80 °C.

### Purification

The pelleted E. coli mass from 10 L culture was re-suspended in 500 mL lysis buffer [50 mM Tris HCl pH 8.0, 300 mM NaCl, 10% (w/w) glycerol, 40 mM imidazol, protease inhibitor cocktail Complete EDTA-free (Roche)], homogenized in a high pressure device (Microfluidics) and afterwards the lysate was centrifuged (100.000 g, 60 min, 4 °C). Several purification steps were performed using an Äkta purification system. The centrifuged soluble lysate fraction was applied in an initial IMAC chromatography step to a column containing 50 mL of Ni-Sepharose HP matrix (GE). Equilibration, fusion protein binding and wash of the Hi-Trap-Sepharose HP matrix was done using Buffer A (50 mM Tris HCl pH 8.0, 300 mM NaCl, 40 mM imidazol). For elution of the NusA-TFPI fusion protein, a linear gradient of imidazol from 40 to 500 mM in Buffer B (50 mM Tris HCl pH 8.0, 150 mM NaCl) was used. The elution fractions were pooled (total volume 140 mL) and applied in fractions to a desalting column Hi Prep 26/10 (GE) (two linked column units) for exchange to a buffer with 50 mM Tris HCl pH 8.0, 150 mM NaCl, 5 mM CaCl₂). For removal of the Nus A tag, a proteolytic digest with His₆-tagged TEV, at an enzyme to fusion protein ratio of 1:66 w/w, was performed for 16 h at 4 °C. The sample was again applied to column containing 50 mL of Ni-Sepharose HP matrix (GE) to separate the free TFPI from uncleaved fusion protein and His-TEV. The eluate of the IMAC step was then applied to size exclusion chromatography, size exclusion chromatography (SEC, column S100, GE) to isolate a monomeric TFPI fraction which was concentrated by ultrafiltration (Amicon, unit with 3 kDa-cut off range) to about 1.5 mg/mL. The purified final TFPI Kunitz domain 1 + 2 sample ran as a double band in PAGE with an apparent molecular weight of about 18 kDa. Further analysis (SEC, western blot) revealed that only protein corresponding to the upper band was immunoreactive with the Fab B.

### Example 3. Proteolytic processing and purification of Fab B from human IgG1.

### Expression

The Fab B was proteolytically processed from its human IgG1 form. Fab B_IgG1 was expressed in mammalian cells (HEK 293) as a secretion protein. For IgG1 isolation, 1.6 L culture supernatant was applied to two linked columns of HiTrap MabSelectSuRE (from GE, 5 mL bed volume, flow rate 1.5 mL/min, 4 °C, for 16 h). For column wash and equilibration, a buffer consisting of PBS and 500 mM NaCl was used. Bound IgG1 was eluted (50 mM Na-acetate, 500 mM NaCl pH 3.5 followed by the same buffer with pH 3.0), neutralized (2.5 M Tris > 11) and concentrated by ultrafiltration to about 13 mg/mL.

Immobilized papain (Pierce, 20 mL slurry) was used for digest of about 270 mg (in 12.5 mL) of IgG1 using 22 fractions in 1.5 mL Eppendorf reaction tubes (incubation 16 h, 37 °C, agitation 1400 rpm). After processing the samples were centrifuged, the supernatant was collected, the pellet washed with PBS and both supernatants and cleared wash were pooled.

The digested sample was again applied to two linked HiTrap MabSelectSuRe columns (2 x 5 mL) enabling a separation of Fc and Fab material. The pooled isolated Fab B fractions were concentrated by ultra filtration to about 8 mg/mL (total yield 120 mg). Additionally, size exclusion chromatography with Superdex75 (column 26/60, flowrate 2.5 mL/min with PBS) was used for further purification. After further concentration and sterile filtration the final yield of the Fab B was 115 mg at a concentration of 8.5 mg/mL.

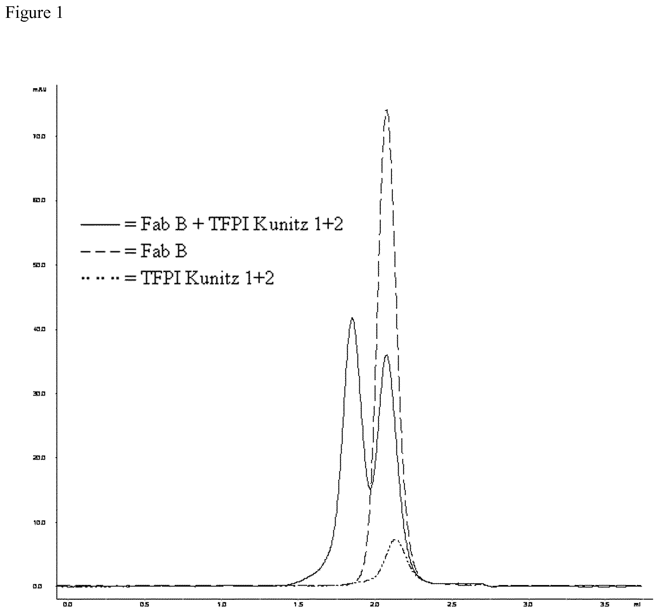

Analytical size exclusion chromatography (Äkta Micro system, S75 5/150 column, 100 mM Tris HCl, pH 7.5) was used to demonstrate Fab B/TFPI KD1+2 complex formation (Figure 1). For Fab B an unexpectedly long retention time on the SEC column was observed corresponding to an apparent molecular weight of 20 kDa, which is very similar to the molecular weight detected for TFPI KD1+2.

### Example 4. Production of the complex TFPI Kunitz domain 1 + 2 with Fab B

In order to form immune complex, TFPI Kunitz domain 1 + 2 and Fab B were combined at a ratio of approximately 1:1.5 (w/w). Therefore, 3.85 mg of the concentrated, monomeric TFPI Kunitz domain 1 + 2 protein (from S100 pooled fractions) was mixed with 7.4 mg Fab B (from SEC Superdex75) and incubated for 16 h at 21 °C. Complex formation was demonstrated via analytical SEC (S200/150) and Western blot. The complex was further purified by SEC (S200 26/26) in 10 mM Tris HCl pH 7.4 with 150 mM NaCl, concentrated by ultrafiltration (Amicon, unit with 5 kDa-cut off range) to 10.3 mg/mL, which was used for crystallization.

### Example 4. Crystallization and X-ray Structure Determination of TFPI-Fab B complex

### Crystallization

Co-crystals of a protein construct comprising TFPI - Kunitz domain 1 (KD1) and Kunitz domain 2 (KD2) and the monoclonal TFPI antibody Fab B were grown at 4 °C using the sitting-drop method. The protein complex was concentrated to 10 mg/mL and crystallized by mixing equal volumes of protein solution and well solution (20% PEG8000) as precipitant. Crystals appeared after three days.

### Data Collection and Processing

Crystals were flash-frozen in liquid nitrogen in 30% glycerol in crystallization buffer for cryo-protection. Data of one crystal was collected at beamline BL14.1, BESSY synchrotron (Berlin) on a MAR CCD detector. Data was indexed and integrated with IMOSFLM (A.G.W. Leslie, (1992), Joint CCP4 + ESF-EAMCB Newsletter on Protein Crystallography, No. 26), prepared for scaling with POINTLESS (P.R. Evans, (2005) Acta Cryst. D62, 72-82), and scaled with SCALA (P.R. Evans, (2005) Acta Cryst. D62, 72-82). The crystal diffracted up to 2.3 Å and possesses space group P2₁ with cell constants *a*=80.3, *b*=71.9, *c*=108.8; *β*=92.5° and two TFPI-KD1, -KD2 - Fab complexes in the asymmetric unit.

### Structure Determination and Refinement

The co-structure of TFPI-KD1, -KD2 and the monoclonal antibody Fab was solved by molecular replacement using PHASER (A.J. McCoy et al (2007) J. Appl. Cryst. 40, 658-674), MOLREP (A.Vagin and A.Teplyakov (1997) J. Appl. Cryst. 30, 1022-10) and in house and published X-ray structures of TFPI-KD2 (pdb code 1tfx) and a Fab fragment (pdb code 1w72) as search models. Prior to molecular replacement Fab and KD1 models were processed with CHAINSAW (N. Stein, (2008) J. Appl. Cryst. 41, 641 - 643). Iterative rounds of model building with COOT (P. Emsley et al. (2010) Acta Cryst. D66:486-501) and maximum likelihood refinement using REFMAC5.5 (G.N. Murshudov et al. (1997) Acta Cryst. D53, 240-255) completed the model. Region hc_Ser131 - hc_Ser136 of both Fabs, TFPI residues Asp1 - Leu21, Asp149 - Phe154, and the KD1 - KD2 linker residues Arg78 - Glu92 showed weak electron density and were not included in the model. Data set and refinement statistics are summarized in Table 1.

**Table 1. Data set and refinement statistics for TFPI-Fab B complex.**

| | |
|---|---|
| Wavelength | 0.9184 Å |
| Resolution (highest shell) | 47-2.3 (2.4-2.3) Å |
| Reflections (observed/unique) | 165457 (56223) |
| Completeness" | 97.8% (97.8%) |
| I/σ*^{a}* | 5.8 (2.0) |
| R_{merge}*^{a,b}* | 0.13 (0.52) |
| Space group | P2₁ |
| Unit cell parameters | |
| *a* | 80.3 Å |
| *b* | 71.9 Å |
| *c* | 108.8 Å |
| *β* | 92.5° |
| R_{cryst}*^{c}* | 0.20 |
| R_{free}*^{d}* | 0.27 |
| Wilson temperature factor | 16.7 Å² |
| RMSD bond length*^{e}* | 0.008 Å |
| RMSD bond angles | 1.3° |
| Protein atoms | 8205 |
| Water molecules | 599 |

| | |
|---|---|
| *^{a}* The values in parentheses are for the high resolution shell. *^{b}* R_{merge} = ∑hkl \|Iₕₖₗ - <Iₕₖₗ>\| / ∑hkl <Iₕₖₗ> where Iₕₖₗ is the intensity of reflection hkl and <Iₕₖₗ> is the average intensity of multiple observations. *^{c}* R_{cryst} = ∑ \|F_{obs}- F_{calc}\| / ∑ F_{obs} where F_{obs} and F_{calc} are the observed and calculated structure factor amplitues, respectively. *^{d}* 5% test set *^{e}* RMSD, root mean square deviation from the parameter set for ideal stereochemistry | |

### Example 5. X-ray Structure-Based Epitope Mapping

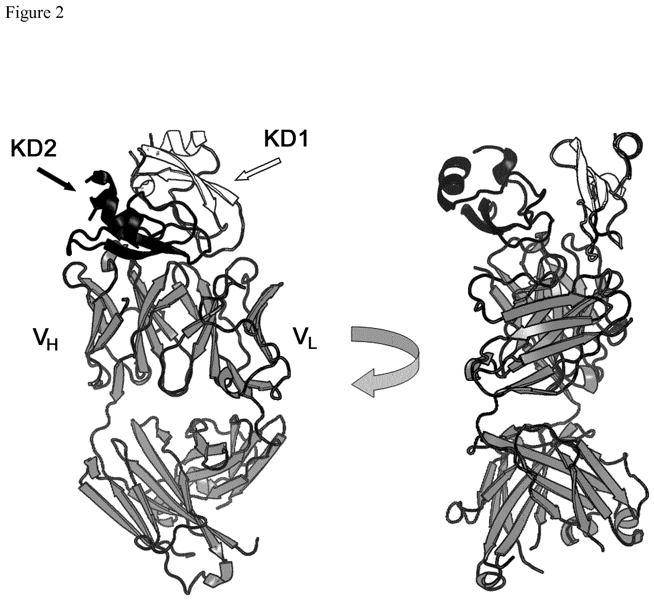

The complex of TFPI-KD1, -KD2, and Fab B (Figure 2) crystallized as two copies of the complex per asymmetric unit. The main chains of the complexes superpose with an overall RMSD of 1.0 Å with each Fab B bound to epitope of the associated TFPI-KD1 and - KD2. Both Kunitz domains interact directly or through water-mediated interactions with Fab B. KD1 and KD2 also interact with each other. Residues of TFPI in contact with Fab B (the epitope) and respective buried surface were analysed with the CCP4 program AREAIMOL (P.J. Briggs (2000) CCP4 Newsletter No. 38). Residues with minimum 5 Å² buried surface or more than 50% buried surface have been considered contacted (Table 2). Residues of Fab B in contact with TFPI (the paratope) and respective buried surface were analysed with AREAIMOL. Residues with minimum of 5 Å² buried surface or more than 50% buried surface have been considered contacted (Table 3).

**Table 2: Residues of TFPI in contact with Fab B. Chains C, D and chains N, O correspond to the TFPI Kunitz domains 1 and Kunitz domain 2 of the respective complex in the asymmetric unit.**

| Residue Nr | buried surface in Å² | buried surface in % |
|---|---|---|
| Phe C 28 | 3.4 | 4.1 |
| Asp C 31 | 26.2 | 47.3 |
| Asp C 32 | 6.8 | 73.9 |
| Gly C 33 | 7.6 | 100.0 |
| Pro C 34 | 87.2 | 97.2 |
| Cys C 35 | 45.7 | 93.4 |
| Lys C 36 | 139.6 | 72.4 |
| Ala C 37 | 0.9 | 2.2 |
| Ile C 38 | 3.0 | 2.1 |
| Cys C 59 | 11.3 | 34.7 |
| Glu C 60 | 83.9 | 60.4 |
| Gly C 61 | 0.5 | 1.4 |
| Asn C 62 | 4.1 | 11.2 |
| Glu D 100 | 83.2 | 53.9 |
| Glu D 101 | 80.8 | 93.4 |
| Pro D 103 | 57.4 | 85.7 |
| Gly D 104 | 16.6 | 68.3 |
| Ile D 105 | 11.4 | 61.2 |
| Cys D 106 | 12.5 | 32.2 |
| Arg D 107 | 116.8 | 73.2 |
| Gly D 108 | 18.6 | 49.4 |
| Tyr D 109 | 133.5 | 74.6 |
| Phe D 114 | 8.5 | 72.0 |
| Asn D 116 | 8.3 | 24.5 |
| Glu D 123 | 45.5 | 56.5 |
| Arg D 124 | 9.9 | 6.0 |
| Phe D 125 | 0.1 | 0.9 |
| Lys D 126 | 29.2 | 39.0 |
| Tyr D 127 | 1.3 | 7.1 |
| Gly D 128 | 6.5 | 67.0 |
| Lys N 29 | 1.1 | 1.2 |
| Asp N 31 | 28.9 | 49.4 |
| Asp N 32 | 10.2 | 91.8 |
| Gly N 33 | 12.5 | 100.0 |
| Pro N 34 | 87.9 | 97.2 |
| Cys N 35 | 42.1 | 86.0 |
| Lys N 36 | 142.5 | 74.2 |
| Ile N 38 | 4.3 | 3.2 |
| Cys N 59 | 11.9 | 38.5 |
| Glu N 60 | 71.7 | 53.0 |
| Gly N 61 | 0.4 | 1.0 |
| Asn N 62 | 7.0 | 20.2 |
| Glu O 100 | 65.1 | 44.3 |
| Glu O 101 | 84.8 | 97.0 |
| Pro O 103 | 60.2 | 84.0 |
| Gly O 104 | 13.6 | 64.7 |
| Ile O 105 | 11.6 | 67.4 |
| Cys O 106 | 12.7 | 37.0 |
| Arg O 107 | 101.3 | 69.1 |
| Gly O 108 | 19.7 | 52.5 |
| Tyr O 109 | 139.6 | 76.4 |
| Thr O 111 | 0.1 | 0.1 |
| Phe O 114 | 11.5 | 78.2 |
| Asn O 116 | 13.4 | 34.6 |
| Glu O 123 | 24.1 | 35.9 |
| Arg O 124 | 11.1 | 6.7 |
| Phe O 125 | 0.1 | 1.2 |
| Lys O 126 | 35.0 | 52.6 |
| Tyr O 127 | 1.2 | 8.1 |
| Gly O 128 | 6.4 | 58.1 |

**Table 3: Residues of Fab B in contact with TFPI. Chains A, B and chains L, M represent the Fab B light and heavy chains of the respective complex in the asymmetric unit.**

| Residue Nr | buried surface in Å² | buried surface in % |
|---|---|---|
| Leu A 27 | 1.8 | 39.1 |
| Arg A 28 | 20.7 | 13.5 |
| Asn A 29 | 44.6 | 38.6 |
| Tyr A 30 | 56.0 | 57.7 |
| Tyr A 31 | 96.9 | 76.7 |
| Tyr A 48 | 43.0 | 75.8 |
| Tyr A 49 | 39.2 | 90.7 |
| Asp A 50 | 16.5 | 56.7 |
| Asn A 52 | 10.3 | 16.8 |
| Pro A 54 | 10.4 | 34.2 |
| Ser A 55 | 5.0 | 3.9 |
| Asn A 65 | 6.3 | 13.4 |
| Trp A 90 | 19.2 | 45.9 |
| Asp A 92 | 13.6 | 10.2 |
| Gly A 93 | 8.7 | 37.8 |
| Gln B 1 | 12.6 | 6.8 |
| Gly B 26 | 29.6 | 58.6 |
| Phe B 27 | 21.1 | 57.9 |
| Thr B 28 | 56.9 | 65.0 |
| Arg B 30 | 32.0 | 19.2 |
| Ser B 31 | 52.2 | 65.9 |
| Tyr B 32 | 54.0 | 94.9 |
| Arg B 52 | 8.0 | 6.2 |
| Arg B 98 | 7.0 | 49.2 |
| Tyr B 100 | 92.2 | 98.6 |
| Arg B 101 | 106.6 | 71.6 |
| Tyr B 102 | 80.2 | 79.4 |
| Trp B 103 | 21.7 | 87.7 |
| Asp B 105 | 15.3 | 42.6 |
| Tyr B 106 | 13.7 | 15.2 |
| Leu L 27 | 4.2 | 61.7 |
| Arg L 28 | 22.2 | 15.5 |
| Asn L 29 | 43.0 | 33.6 |
| Tyr L 30 | 58.2 | 67.3 |
| Tyr L 31 | 103.4 | 80.3 |
| Tyr L 48 | 48.7 | 83.8 |
| Tyr L 49 | 37.5 | 88.8 |
| Asp L 50 | 15.5 | 59.1 |
| Asn L 52 | 8.6 | 14.8 |
| Pro L 54 | 12.9 | 45.4 |
| Ser L 55 | 9.8 | 8.0 |
| Asn L 65 | 3.9 | 7.7 |
| Gly L 67 | 0.1 | 0.3 |
| Trp L 90 | 20.3 | 48.3 |
| Asp L 92 | 1.9 | 1.5 |
| Gly L 93 | 18.4 | 49.4 |
| Val M 2 | 1.6 | 4.3 |
| Gly M 26 | 34.2 | 62.2 |
| Phe M 27 | 18.2 | 62.1 |
| Thr M 28 | 64.4 | 69.7 |
| Arg M 30 | 27.1 | 18.8 |
| Ser M 31 | 51.5 | 63.7 |
| Tyr M 32 | 55.3 | 95.3 |
| Arg M 52 | 7.4 | 6.2 |
| Arg M 98 | 8.5 | 57.0 |
| Tyr M 100 | 86.9 | 98.3 |
| Arg M 101 | 110.8 | 74.4 |
| Tyr M 102 | 82.8 | 81.0 |
| Trp M 103 | 18.5 | 91.1 |
| Asp M 105 | 17.3 | 48.7 |
| Tyr M 106 | 13.5 | 15.0 |

The Fab B recognized a non-linear epitope of KD1 and KD2 which is defined by residues Asp31 - Lys36, Cys59 (which forms a disulfide bridge with Cys35), Glu60, and Asn62 of TFPI-KD1 and Glu100, Glu101, region Pro103 - Cys106 (which forms a disulfide bridge with Cys130), residues Arg107- Tyr109, Phe114, Asn116, Glu123, Arg124, and residues Lys126 - Gly128 of TFPI-KD2. The paratope in Fab B which interacts with TFPI-KD1 includes lc_Leu27 - lc_Tyr31, lc_Asp50, lc_Asn65, lc_Trp90, lc_Asp92, lc_Gly93, and hc_Arg101 and hc_Tyr102. The paratope in Fab B which interacts with TFPI-KD2 includes lc_Tyr31, lc_Tyr48 and lc_Tyr49, and hc_Thr28, hc_Arg30 - hc_Tyr32, hc_Tyr100, hc_Arg101, hc_Trp103, and hc_Asp105. The light chain CDRs appear to be the major interaction sites for TFPI-KD1, the heavy chain CDRs appear to be the major interaction sites for TFPI-KD2, based on the number of contacts.

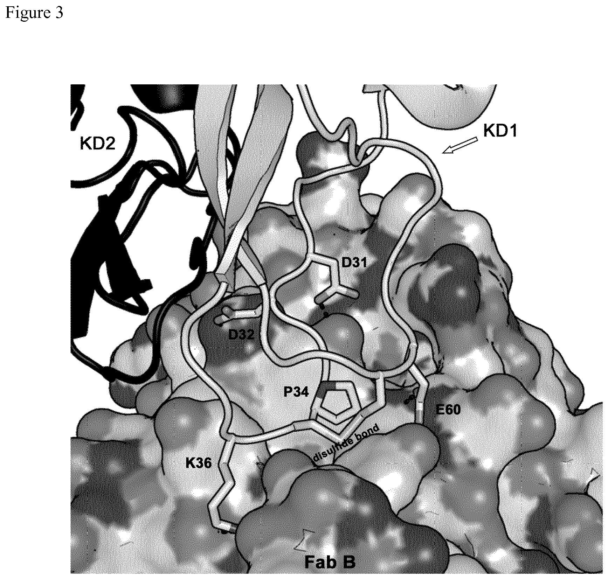

The non-linear epitope on TFPI-KD1 consists of two loop regions linked by a disulfide bridge between Cys35 and Cys59. The epitope is characterized by a central hydrophobic interaction of Pro34 surrounded by a triangle of polar interactions of Asp31, Asp32, Glu60, and Lys36 with Fab B (Figure 3).

Pro34 lies in a hydrophobic cleft created by lc_Tyr30 and lc_Tyr31 of CDR-L1, lc_Trp90 of CDR-L3 and hc_Tyr102 of CDR-H3. Asp31 and Asp32 possess polar interaction with CDR-H3 and a hydrogen bond network with hc_Arg101, hc_Tyr102, and a water molecule. Hc_Tyr102 side chain is well oriented to possess hydrophobic interaction with Pro34, polar interaction with Asn31, and aromatic π-π interaction with lc_Trp90 of CDR-L3.

Interaction of Asp31 and Asp32 with CDR-H3 is a key epitope feature and orientations and interactions of hc_Tyr102 and hc_Arg101 appear crucial. Mutation of *wild type* residue hc_Lys99 to leucine resulted in 20 fold affinity increase. Hc_Leu99 is located in the hydrophobic interface between light and heavy chain and followed by the CDR-H3 loop. A polar and flexible lysine side chain is a disadvantage at this position and interferes with optimal CDR-H3 conformation and antigen interactions.

Glu60, which forms the second corner of the polar triangle, interacts with the side chains of lc_Tyr30 (CDR-L1), lc_Trp90 and main chain nitrogen of lc_Gly93 (CDR-L3).

The third corner of the triangle is formed by Lys36. Lys36 is an essential residue for inhibition of the factor VIIa/tissue factor complex by TFPI (M.S. Bajaj et al. (2001) Thromb Haemost 86(4):959-72.). In complex with Fab B, Lys36 is significantly contacted and buried by CDR-L1 lc_Leu27, lc_Arg28, lc_Asn29, lc_Tyr31, CDR-L2 lc_Asp50, and a water molecule. Interaction of Lys36 with factor VIIa/tissue factor complex while bound to Fab B and its inhibition appear excluded.

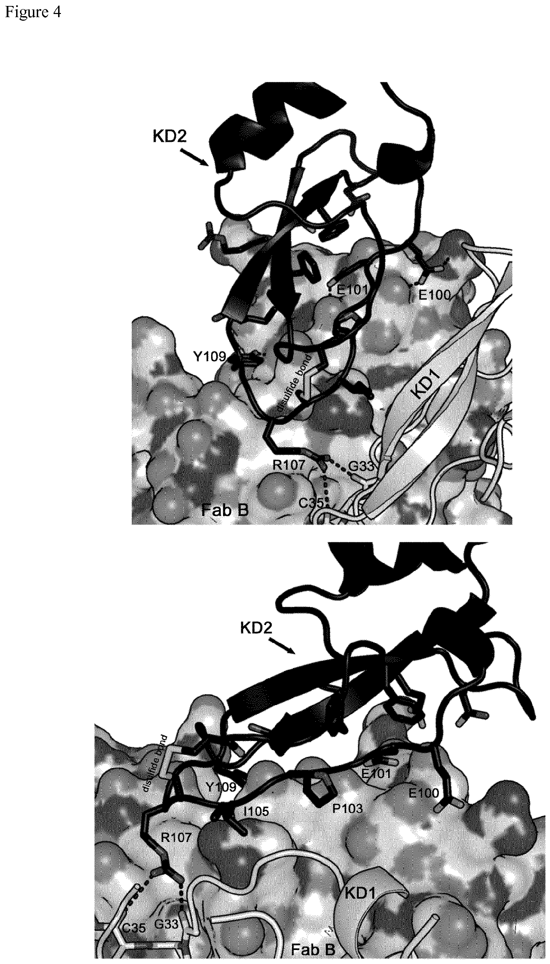

The non-linear epitope on TFPI-KD2 consists of three sections comprising residues Glu100, Glu101, Pro103 - Tyr109, Phe114; Asn116 and Glu123; Arg124, Lys126 - Gly128. The KD2-epitope forms polar and hydrophobic interactions with Fab B CDR-L1, - L2, -H1, and -H3 (Figure 4).

Glu100, Glu101, Arg107, and Tyr109 are key epitope residues providing strong polar or hydrophobic anchor points in contact with three separated surface regions of Fab B created by CDR-H1 (interaction with Glu100 and Glu101) CDR-L1, -L2, -H3 (interaction with Arg107), and CDR-L2, -H3 (interaction with Tyr109).

Arg107 is significantly contacted by lc_Tyr31, lc_Tyr49, hc_Arg101, and hc_Tyr102 of CDR-L1, -L2, -H3, respectively, and additionally interacts with Gly33 and Cys35 of KD1. Arg107 has been shown to be essential for inhibition of factor Xa (M.S. Bajaj et al. (2001) Thromb Haemost 86(4):959-72.). Fab B occupies this critical residue and excludes Arg107 function in inhibiting factor Xa.

Glu100 and Glu101 form hydrogen bonds with CDR-H1 residues hc_Arg30, hc_Ser31, and hc_Thr28 and hc_Tyr32.

Tyr109 lies in a hydrophobic niche created by CDR-L2 lc_Tyr48, and CDR-H3 residues hc_Tyr100, and hc_Trp103, and forms a hydrogen bond with hc_Asp105.

### Example 6. Paratope comparison of Fab B and its optimized variant Fab D

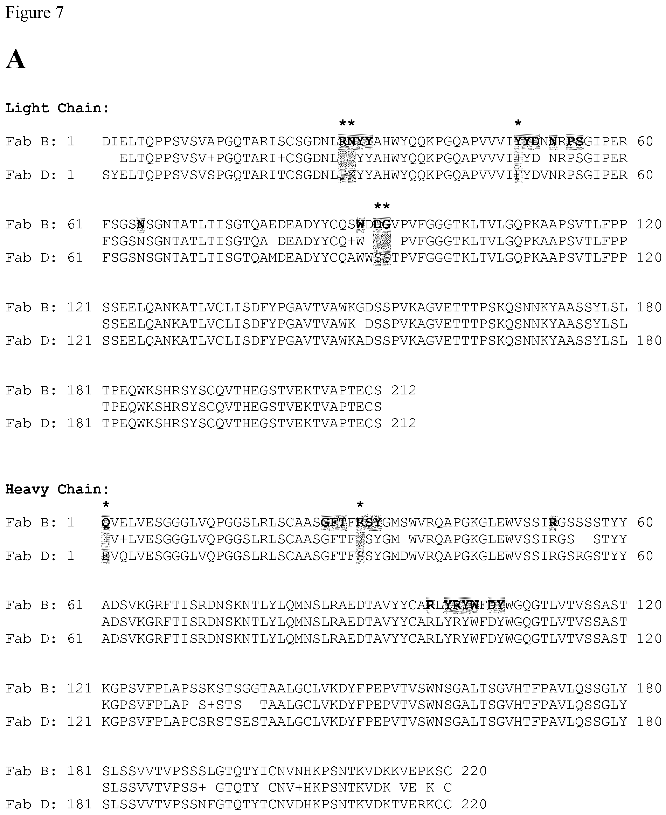

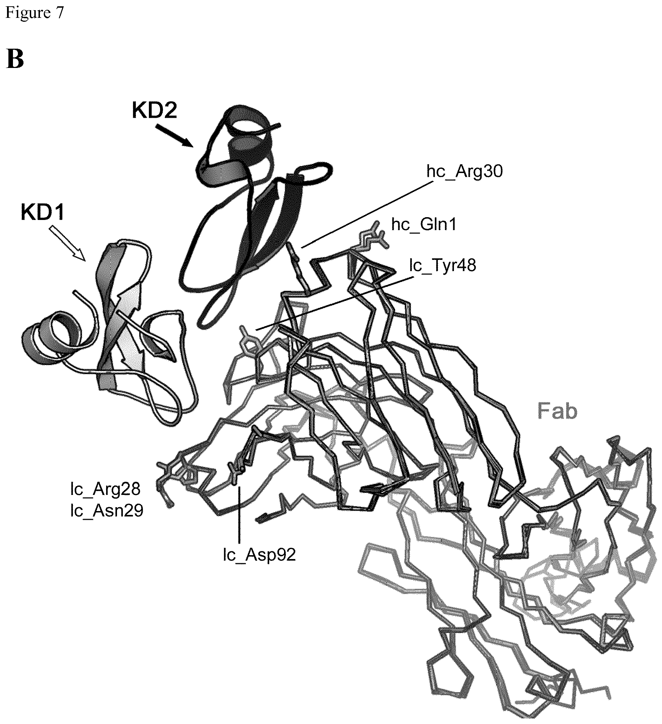

To assess consistency of TFPI epitope binding by the optimized variant of Fab B, Fab D, sequence alignments of the light and heavy chains (Figure 7A) and homology models of Fab D (Figure 7B) were analysed for conservation of Fab B paratope residues in Fab D. Homology models were calculated with DS MODELER (ACCELRYS, Inc; Fiser, A. and Sali A. (2003) Methods in Enzymology, 374:463-493) using our TFPI - Fab B X-ray structure as input template structure. The homology models show nearly identical backbone conformations in comparison to Fab B with RMSD < 0.5 Å. Of 29 paratope residues observed in TFPI-Fab B complex, seven residues (five light chain residues, two heavy chain residues) differ in Fab D (Figure 7). Lc_Arg28; lc_Asn29, lc_Asp92, and lc_Gly93 show main chain interactions with the TFPI epitope residues. The exchanges of these residues in Fab D are not expected to induce binding to a different TFPI epitope. The replacement of lc_Tyr48 and hc_Gln1 by a phenylalanine and glutamate in Fab D are negligible as no polar side chain interactions are observed in the X-ray structure. Hc Arg30 shows a polar interaction with Glu100 of TFPI and is exchanged to a serine in Fab D. At this position, an arginine should be favorable over a serine to interact with TFPI. Based on expected impact of the analysed exchanges between Fab B and Fab D paratope residues, overall sequence conservation and low RMSD of Fab D homology model, Fab D is contemplated to recognize the same TFPI epitope as Fab B.

### Example 7 X-ray Structure-based Rationale for Inhibition of TFPI Interaction with-Factor Xa and Factor VIIa/Tissue Factor complex

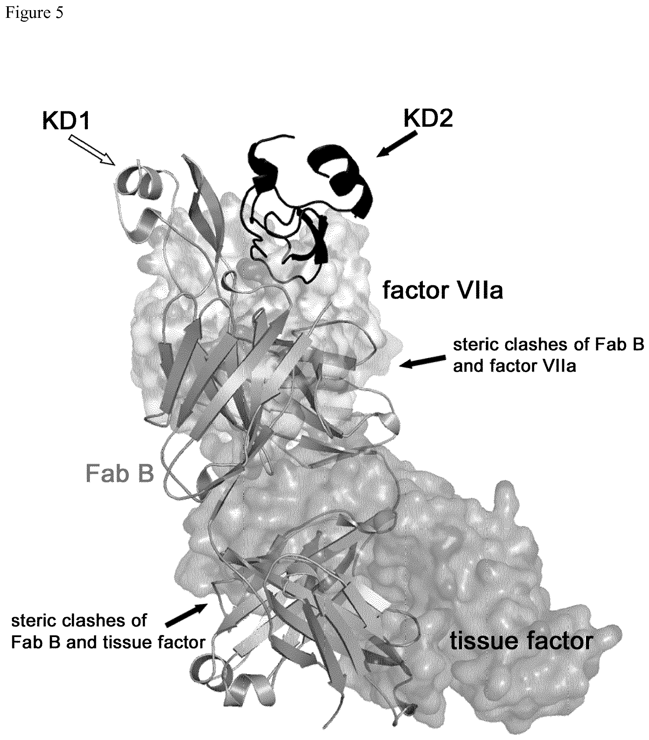

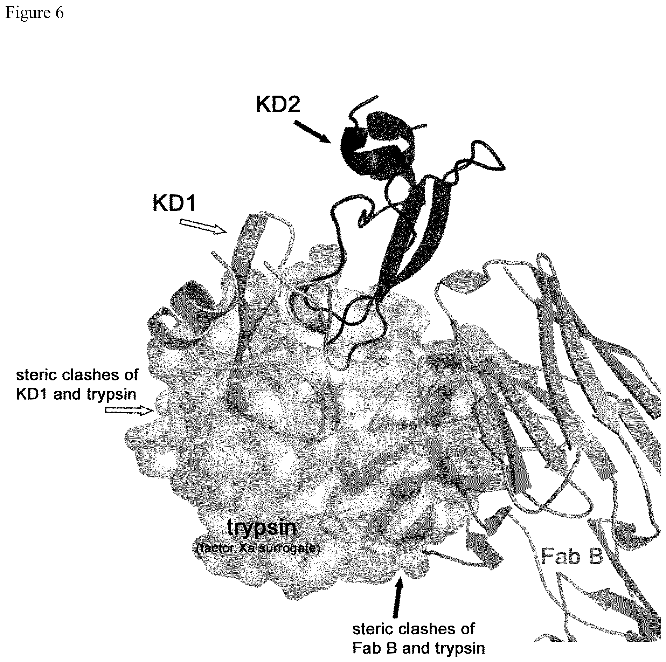

Fab B binds to both KD1 and KD2 of TFPI. KD2 binds and inhibits factor Xa. KD1 binds and inhibits factor VIIa/tissue factor complex. The X-ray structures of KD2 in complex with trypsin (M.J. Burgering et al (1997) J Mol Biol. 269(3):395-407) and BPTI in complex with an extracellular portion of TF and factor VIIa (E. Zhang et al (1999) J Mol Biol 285(5):2089-104.) have been reported. Trypsin is a surrogate for factor Xa, BPTI is a homolog of TFPI-KD1. Superposition of the TFPI-Fab B complex with either KD2-trypsin or BPTI-factor VIIa/tissue factor reveals that antibody binding excludes binding of KD1 and KD2 to their natural ligands factor VIIa/tissue factor and factor Xa, respectively (Figure 5, Figure 6).

## Claims

1. An isolated monoclonal antibody that binds to an epitope of human tissue factor pathway inhibitor (SEQ ID NO: 1),
wherein said epitope comprises one or more residues of Kunitz domain 1, selected from the group consisting of Asp31, Asp32, Gly33, Pro34, Cys35, Lys36, Cys59, Glu60 and Asn62, and
wherein said epitope comprises one or more residues of Kunitz domain 2, selected from the group consisting of Glu100, Glu101, Pro103, Gly104, Ile105, Cys106, Arg107, Gly108, Tyr109, Phe114, Asn116, Glu123, Arg124, Lys126, Tyr127 and Gly128 and wherein the isolated monoclonal antibody comprises a variable light chain (V_{L}) of SEQ ID NO: 4 and a variable heavy chain (V_{H}) of SEQ ID NO: 5.

2. A pharmaceutical composition comprising a therapeutically effective amount of the monoclonal antibody of claim 1 and a pharmaceutically acceptable carrier.

## Patentansprüche

1. Isolierter monoklonaler Antikörper, der an ein Epitop von menschlichem TFPI (Tissue Factor Pathway Inhibitor) (SEQ ID NO: 1) bindet,
wobei das Epitop einen oder mehrere Reste von Kunitz-Domäne 1 umfasst, die aus der Gruppe bestehend aus Asp31, Asp32, Gly33, Pro34, Cys35, Lys36, Cys59, Glu60 und Asn62 ausgewählt sind, und
wobei das Epitop einen oder mehrere Reste von Kunitz-Domäne 2 umfasst, die aus der Gruppe bestehend aus Glu100, Glu101, Pro103, Gly104, Ile105, Cys106, Arg107, Gly108, Tyr109, Phe114, Asn116, Glu123, Arg124, Lys126, Tyr127 und Gly128 ausgewählt sind,
und wobei der isolierte monoklonale Antikörper eine variable leichte Kette (V_{L}) unter SEQ ID NO: 4 und eine variable schwere Kette (V_{H}) unter SEQ ID NO: 5 umfasst.

2. Pharmazeutische Zusammensetzung, umfassend eine therapeutisch wirksame Menge des monoklonalen Antikörpers nach Anspruch 1 und einen pharmazeutisch unbedenklichen Träger.

## Revendications

1. Anticorps monoclonal isolé qui se lie à un épitope d'inhibiteur de voie de facteur tissulaire humain (SEQ ID NO: 1),
dans lequel ledit épitope comprend un ou plusieurs résidus de domaine Kunitz 1, choisis dans le groupe constitué d'Asp31, Asp32, Gly33, Pro34, Cys35, Lys36, Cys59, Glu60 et Asn62, et
dans lequel ledit épitope comprend un ou plusieurs résidus de domaine Kunitz 2, choisis dans le groupe constitué de Glu100, Glu101, Pro103, Gly104, Ile105, Cys106, Arg107, Gly108, Tyr109, Phe114, Asn116, Glu123, Arg124, Lys126, Tyr127 et Gly128
et dans lequel l'anticorps monoclonal isolé comprend une chaîne légère (V_{L}) variable de SEQ ID NO: 4 et une chaîne lourde (V_{H}) variable de SEQ ID NO: 5.

2. Composition pharmaceutique comprenant une quantité thérapeutiquement efficace de l'anticorps monoclonal selon la revendication 1 et un véhicule pharmaceutiquement acceptable.
